# EUROPÄISCHE PATENTSCHRIFT

(11) **EP 3 538 174 B1**
(45) Veröffentlichungstag und Bekanntmachung des Hinweises auf die Patenterteilung: **29.07.2020**
(21) Anmeldenummer: 17791416.5
(22) Anmeldetag: 30.10.2017
(51) Int. Cl.: A61M 1/14, A61M 39/10, A61M 1/16

(54) **VERBINDUNGSSTÜCK FÜR DIE HERSTELLUNG EINER FLÜSSIGKEITSVERBINDUNG ZWISCHEN FLÜSSIGKEITSFÜHRENDEN LEITUNGEN UND MEDIZINISCHES GERÄT MIT EINEM DERARTIGEN VERBINDUNGSSTÜCK**
CONNECTION PIECE FOR PRODUCING A LIQUID CONNECTION BETWEEN LIQUID-CONVEYING LINES, AND MEDICAL APPLIANCE WITH SUCH A CONNECTION PIECE
RACCORD POUR LA RÉALISATION D'UNE LIAISON FLUIDIQUE ENTRE DES CONDUITES GUIDANT UN LIQUIDE ET APPAREIL MÉDICAL PRÉSENTANT UN TEL RACCORD

(30) Priorität: 10.11.2016 DE 102016013334
(43) Veröffentlichungstag der Anmeldung: 18.09.2019
(73) Patentinhaber: Fresenius Medical Care Deutschland GmbH, 61352 Bad Homburg v.d.H (DE)
(72) Erfinder: HÜMMER, Dirk, 97520 Hirschfeld (DE); NICHOLAS, Olaf, 97318 Kitzingen (DE); ASCHENBRENNER, Paul, 97453 Schonungen (DE)
(74) Vertreter: Oppermann, Frank
(86) Internationale Anmeldenummer: PCT/EP2017/077829
(87) Internationale Veröffentlichungsnummer: WO 2018/086950

(56) Entgegenhaltungen:
- WO-A1-2004/108206
- WO-A1-2009/044220
- WO-A1-2013/135386
- DE-A1-102013 107 323

## Beschreibung

Die Erfindung betrifft ein Verbindungsstück für die Herstellung einer Flüssigkeitsverbindung von flüssigkeitsführenden Leitungen zur Montage in einer Ausnehmung eines Gehäuseteils eines medizinischen Geräts, insbesondere Dialysegeräts. Darüber hinaus betrifft die Erfindung ein medizinisches Gerät, insbesondere Dialysegerät, mit einem Gehäuse, das einen Gehäuseteil mit einer Ausnehmung aufweist, in welche ein derartiges Verbindungsstück eingesetzt ist.

Es sind medizinische Geräte bekannt, die über einen Flüssigkeitsteil verfügen, der flüssigkeitsführende Leitungen aufweist. Die Verbindung bzw. der Anschluss der flüssigkeitsführenden Leitungen erfolgt mit Verbindungsstücken, an die sich die Leitungen anschließen lassen. Für die Stromversorgung verfügen die medizinischen Geräte über ein Netzteil, das einen zentralen Schutzleiteranschluss aufweist, der mit dem Schutzleitersystem der festen Elektroinstallation verbunden wird, das sich auf Erdpotential befindet. Die Prüfung elektrischer Geräte schließt die Prüfung des elektrischen Widerstands der Schutzleiterverbindung ein.

Die bekannten Dialysegeräte verfügen über einen extrakorporalen Blutkreislauf und einen Dialysierflüssigkeitsteil. Der extrakorporale Blutkreislauf umfasst die Blutkammer und der Dialysierflüssigkeitsteil die Dialysierflüssigkeitskammer eines Dialysators, der durch eine semipermeable Membran in die beiden Kammer unterteilt ist. Der Dialysator ist im Allgemeinen eine austauschbare Einheit, die an der Außenseite des Gehäuses des Dialysegeräts leicht zugänglich befestigt ist.

Die Dialysegeräte können über eine Einrichtung zur Herstellung von Dialysierflüssigkeit aus Frischwasser und Konzentraten verfügen, die sich innerhalb des Gehäuses befindet. Die frische Dialysierflüssigkeit wird der Dialysierflüssigkeitskammer von der Einrichtung zur Herstellung von Dialysierflüssigkeit über eine Dialysierflüssigkeitszuführleitung zugeführt und aus der Dialysierflüssigkeitskammer wird gebrauchte Dialysierflüssigkeit über eine Dialysierflüssigkeitsabführleitung abgeführt. Da sich der Dialysator nicht in dem Gehäuse des Dialysegeräts befindet, müssen die Dialysierflüssigkeitsleitungen einen Gehäuseteil des Dialysegeräts durchdringen. Für den Anschluss der entsprechenden Schlauchleitungsabschnitte finden Verbindungs- oder Anschlusstücke Verwendung, die auch als Tüllen bezeichnet werden.

Die bekannten Verbindungsstücke werden in passende Ausnehmungen eines Gehäuseteils des Dialysegeräts eingesetzt. Der Gehäuseteil mit der Ausnehmung besteht im Allgemeinen aus einem elektrisch leitfähigen Material (Metallgehäuse). Die Verbindungsstücke weisen einen in die Ausnehmung des Gehäuseteils einsetzbaren rohrförmigen Körper auf, dessen Endstücke zum Anschluss der flüssigkeitsführenden Leitungen ausgebildet sind. Im Allgemeinen besteht der rohrförmige Körper der Verbindungstücke aus einem elektrisch leitfähigen Material, so dass eine elektrische Verbindung zwischen der Dialysierflüssigkeit und dem Metallgehäuse des Dialysegeräts hergestellt wird. Es sind aber auch Verbindungsstücke aus einem nicht elektrisch leitenden Material bekannt, die aber über metallische Einsatzstücke verfügen.

Ein Verbindungsstück für die Herstellung einer Flüssigkeitsverbindung von flüssigkeitsführenden Leitungen zur Montage in einer Buchsen-Einheit eines Gehäuseteils eines medizinischen Geräts, insbesondere eines Dialysegeräts, ist beispielsweise aus der WO 2013/135386 A1 bekannt.

Der Erfindung liegt die Aufgabe zugrunde, ein Verbindungsstück für die Herstellung einer Flüssigkeitsverbindung von flüssigkeitsführenden Leitungen zu schaffen, das sich einfach und sicher in einer Ausnehmung eines Gehäuseteils eines medizinischen Geräts, insbesondere Dialysegeräts, montieren lässt. Insbesondere liegt der Erfindung die Aufgabe zugrunde, ein Verbindungsstück zu schaffen, das einerseits eine feste mechanische Verbindung mit dem Gehäuse und andererseits eine von der mechanischen Verbindung unabhängige elektrische Verbindung mit einem Schutzleiter oder einem Potentialausgleich erlaubt.

Eine weitere Aufgabe der Erfindung liegt darin, ein medizinisches Gerät, insbesondere Dialysegerät, zu schaffen, das eine sichere Prüfung des Widerstandes der Schutzleiterverbindung erlaubt.

Die Lösung dieser Aufgaben erfolgt erfindungsgemäß mit den Merkmalen der unabhängigen Patentansprüche. Die abhängigen Ansprüche betreffen vorteilhafte Ausführungsformen der Erfindung.

Das erfindungsgemäße Verbindungsstück für die Herstellung einer Flüssigkeitsverbindung von flüssigkeitsführenden Leitungen, das zur Montage in einer Ausnehmung eines Gehäuseteils eines medizinischen Geräts, insbesondere Dialysegeräts, bestimmt ist, weist einen in die Ausnehmung des Gehäuseteils einsetzbaren rohrförmigen Körper aus einem elektrisch leitfähigen Material auf, dessen Endstücke zum Anschluss der flüssigkeitsführenden Leitungen ausgebildet sind. Die Endstücke können als Anschlussstücke ausgebildet sein, an denen Schlauchleitungen angeschlossen werden können. Beispielsweise können die Schlauchleitungen einfach auf die Endstücke aufgeschoben werden. Es ist aber auch möglich, dass die Endstücke über Stecker oder Buchsen oder andere Konnektoren verfügen.

Das erfindungsgemäße Verbindungsstück zeichnet sich durch einen Anschlussteil zum Anschluss eines elektrischen Schutzleiters oder Potentialausgleichs aus, wobei der Anschlussteil elektrisch leitend mit dem rohrförmigen Körper verbindbar ist oder verbunden ist. Der Anschlussteil ermöglicht einen direkten Anschluss einer elektrischen Verbindungsleitung an einen Schutzleiter (PE-Leiter) oder einen Potentialausgleich. Von Vorteil ist, dass der Schutzleiteranschluss von der mechanischen Verbindung des Verbindungsstücks mit dem Gehäuseteil unabhängig ist. Selbst wenn sich die mechanische Verbindung lösen sollte, bleibt die für die elektrische Sicherheit des medizinischen Geräts erforderliche Erdung bestehen.

Eine bevorzugte Ausführungsform des erfindungsgemäßen Verbindungsstücks sieht vor, dass der Anschlussteil ein Ringkabelschuh ist, der mit dem rohrförmigen Körper verschraubt werden kann. Der rohrförmige Körper weist vorzugsweise einen umlaufenden Ansatz auf, an den sich ein Außengewinde anschließt. Wenn der Ringkabelschuh mit einer auf das Außengewinde aufgeschraubten Mutter verschraubt wird, kann der Ringkabelschuh zwischen dem umlaufenden Ansatz und der Mutter sicher verspannt werden.

Der Anschlussteil kann aber auch eine Anschlussfahne sein, die mit dem rohrförmigen Körper elektrisch leitend verbunden ist. Die Anschlussfahne kann beispielsweise mit dem rohrförmigen Körper verlötet sein. Bei dieser Ausführungsform entfällt also eine Schraubverbindung.

Bei einer besonders bevorzugten Ausführungsform weist der rohrförmige Körper zwischen den Endstücken einen Flansch, mit dem sich der rohrförmige Körper an dem Gehäuseteil abstützen kann, und einen Sicherungsteil auf, der an dem rohrförmigen Körper befestigt werden kann, so dass der rohrförmige Körper in der Ausnehmung des Gehäuseteils in axialer Richtung gesichert ist. Dadurch ist das Verbindungsstück sicher mit dem Gehäuseteil mechanisch verbunden.

Bei einer Ausführungsform, bei der das Gehäuse des medizinischen Geräts aus einem elektrisch leitenden Material besteht, was im Allgemeinen der Fall ist, verfügt der Flansch des rohrförmigen Körpers über einen inneren Flanschteil, der mit dem rohrförmigen Körper einstückig ist, und einen äußeren Flanschteil, der eine Ausnehmung aufweist, in die der innere Flanschteil eingesetzt werden kann. Der rohrförmige Körper kann sich dann mit dem äußeren Flanschteil an dem Gehäuseteil abstützen. Da der äußere Flanschteil aus einem elektrisch nicht leitenden Material besteht, wird der rohrförmige Körper gegenüber dem Gehäuseteil aus elektrisch leitendem Material isoliert. Folglich besteht zwischen dem Anschlussteil und dem Gehäuse keine elektrische Verbindung. Dies ist insofern von Vorteil als bei einer Prüfung der Schutzleiterverbindung der Widerstand zwischen dem Anschlussteil und dem Schutzleiteranschluss direkt gemessen werden kann, ohne dass eine andere elektrische Verbindung, beispielsweise über andere Gehäuseteile, das Messergebnis verfälschen könnte.

Wenn der Gehäuseteil des medizinischen Geräts aber aus einem elektrisch nicht leitenden Material bestehen sollte, beispielsweise ein Kunststoffgehäuse sein sollte, ist eine zweiteilige Ausbildung des Flansches mit einem Isolierstück nicht erforderlich.

Bei einer weiteren besonders bevorzugten Ausführungsform ist der innere Flanschteil in dem äußeren Flanschteil gegen Verdrehen gesichert. Dadurch wird sichergestellt, dass ein an dem Anschlussteil angeschlossenes elektrisches Verbindungskabel nicht durch Verdrehen des Verbindungsstücks in dem Gehäuseteil abreißen kann.

Die Sicherung gegenüber Verdrehen kann dadurch erfolgen, dass die beiden Bauteile einen nicht runden Querschnitt haben. Der äußere Flanschteil weist vorzugsweise eine mehreckige Ausnehmung und der innere Flanschteil eine mehreckige Umfangsfläche auf.

Der Sicherungsteil ist vorzugsweise ein Sicherungsring, der auf den rohrförmigen Körper einfach aufgesetzt werden kann. Der Sicherungsring besteht vorzugsweise aus Metall. Durch Zwischenlage einer Unterlegscheibe aus einem elektrisch nicht leitenden Material zwischen dem metallischen Sicherungsring, der elektrisch mit dem rohrförmigen Körper verbunden ist, und dem Flanschteil des rohrförmigen Körpers, kann der rohrförmige Körper gegenüber dem Gehäuseteil isoliert werden. Der Sicherungsring kann aber auch aus Kunststoff bestehen. Dann kann eine zusätzliche Unterlegscheibe aus Kunststoff zur elektrischen Isolation entfallen. Die Verwendung eines Sicherungsrings aus Kunststoff setzt aber voraus, dass dieser den mechanischen Ansprüchen genügt, so dass das Verbindungsstück verdrehsicher, elektrisch isoliert mit dem Gehäuse mechanisch verbunden ist.

Das erfindungsgemäße medizinische Gerät weist ein Netzteil mit einem zentralen Schutzleiteranschluss auf, der mit einem elektrischen Verbindungskabel mit dem Anschlussteil des Verbindungsstücks elektrisch verbunden ist. Das erfindungsgemäße Verbindungsstück ist für ein medizinisches Gerät bestimmt, dass einen Flüssigkeitsteil mit flüssigkeitsführenden Leitungen aufweist, wobei die flüssigkeitsführenden Leitungen an den Endstücken des Verbindungsstücks angeschlossen sind. Das medizinisches Gerät ist insbesondere ein Dialysegerät, das einen Dialysierflüssigkeitsteil mit einer zu einem Dialysator führenden Dialysierflüssigkeitszuführleitung und einer von dem Dialysator abgehenden Dialysierflüssigkeitsabführleitung aufweist, wobei ein Abschnitt der Dialysierflüssigkeitszuführleitung oder Dialysierflüssigkeitsabführleitung an dem einen Endstück und ein anderer Abschnitt der Dialysierflüssigkeitszuführleitung oder Dialysierflüssigkeitsabführleitung an dem anderen Endstück des Verbindungstücks angeschlossen sind.

Im Folgenden wird ein Ausführungsbeispiel der Erfindung unter Bezugnahme auf die Zeichnungen im Einzelnen beschrieben.

Es zeigen:
- Fig. 1: einen Teil des Gehäuses eines Dialysegeräts mit den erfindungsgemäßen Verbindungsstücken in stark vereinfachter schematischer Darstellung,
- Fig. 2: das erfindungsgemäße Verbindungsstück in der Seitenansicht,
- Fig. 3: das Verbindungsstück in geschnittener Darstellung,
- Fig. 4: eine Explosionsdarstellung des Verbindungsstücks und
- Fig. 5: den Teil A des Verbindungstücks von Fig. 4 in vergrößerter Darstellung .

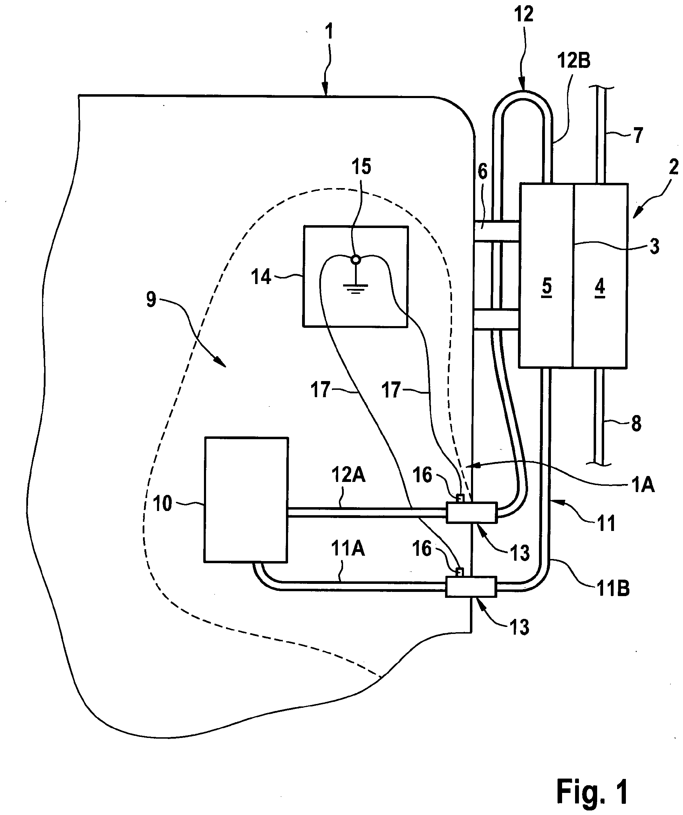

Fig. 1 zeigt die für die Erfindung wesentlichen Komponenten eines Dialysegeräts als Beispiel für ein medizinisches Gerät in stark vereinfachter schematischer Darstellung. Das Dialysegerät weist ein Gehäuse 1 auf, das aus einem elektrisch leitfähigen Material besteht (Metallgehäuse). Das Gehäuse 1 kann aus mehreren Gehäuseteilen bestehen.

Das Dialysegerät weist einen in Fig. 1 nur schematisch dargestellten Dialysator 2 auf, der durch eine semipermeable Membran 3 in eine Blutkammer 4 und eine Dialysierflüssigkeitskammer 5 unterteilt ist. Der Dialysator 2 kann an einer Halterung 6 befestigt werden, die an der Außenseite des Gehäuses 1 vorgesehen ist. Zu der Blutkammer 4 des Dialysators 2 führt eine Blutzuführleitung 7 und eine Blutabführleitung 8 geht von der Blutkammer 4 ab. Die Blutkammer 4 und die Blutzuführ- und Blutabführleitung 7, 8 bilden den extrakorporalen Blutkreislauf.

Das Dialysegerät verfügt über einen Dialysierflüssigkeitsteil 9, der eine Einrichtung 10 zur Herstellung von Dialysierflüssigkeit aus Frischwasser und Konzentraten aufweist. Von der Einrichtung 10 zur Herstellung von Dialysierflüssigkeit führt eine Dialysierflüssigkeitszuführleitung 11 zu einem Einlass der Dialysierflüssigkeitskammer 5 und von einem Auslass der Dialysierflüssigkeitskammer 5 führt eine Dialysierflüssigkeitsabführleitung 12 zu dieser Einrichtung 10 oder einen Auslauf. Die Dialysierflüssigkeitsleitungen 10, 11 weisen jeweils zwei Leitungsabschnitte 11A, 11B, 12A, 12B auf, die an den erfindungsgemäßen Verbindungsstücken 13 angeschlossen sind, die in Fig. 1 nur schematisch dargestellt sind.

Darüber hinaus weist das Dialysegerät ein Netzteil 14 auf, das in dem Gehäuse 1 einen zentralen Schutzleiteranschluss 15 oder Schutzleiterkontakt hat. Die Verbindungsstücke 13 weisen einen in Fig. 1 nur schematisch dargestellten Anschlussteil 16 auf, der mit einem elektrischen Verbindungskabel 17 mit dem zentralen Schutzleiteranschluss 15 verbunden ist. Beim Anschluss des Netzteils 14 an das Netz wird eine elektrische Verbindung zwischen dem zentralen Schutzleiteranschluss 15 und dem Schutzleitersystem der festen Elektroinstallation hergestellt.

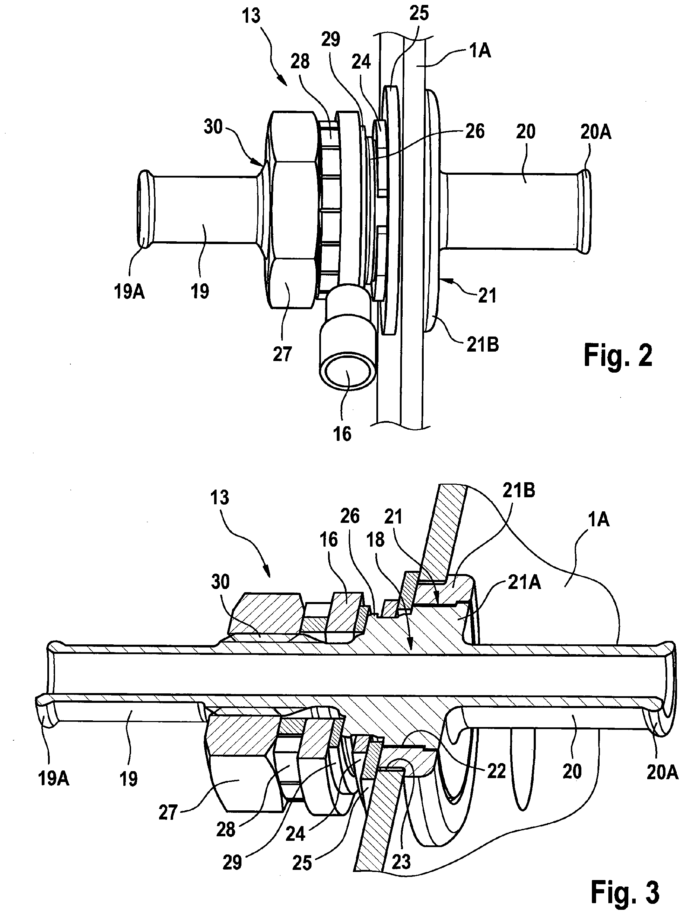

Nachfolgend wird ein Ausführungsbeispiel des erfindungsgemäßen Verbindungsstücks 13 unter Bezugnahme auf die Figuren 2 bis 5 im Einzelnen beschrieben. Dabei wird auf das Gehäuseteil 1A Bezug genommen, an dem die erfindungsgemäßen Verbindungsstücke 13 vorgesehen sind. Fig. 2 zeigt eine Seitenansicht und Fig. 3 zeigt einen Schnitt durch das Verbindungsstück.

Das Verbindungsstück 13 weist einen rohrförmigen Körper 18 aus einem metallischen Material, beispielsweise Edelstahl, auf. Die beiden Endstücke 19, 20 des rohrförmigen Körpers sind als Anschlussstücke für die Leitungsabschnitte 11A, 11B, 12A, 12B der Dialysierflüssigkeitsleitungen 11, 12 ausgebildet. Sie weisen an den Enden einen umlaufenden Wulst 19A, 20A auf, so dass sich die Schlauchleitungen aufschieben lassen und gegen Abziehen gesichert sind. Zur sicheren Befestigung können noch Schlauchklemmen oder dgl. vorgesehen sein.

Der rohrförmige Körper 18 weist einen zweiteiligen Flansch 21 auf, der einen inneren Flanschteil 21A, der mit dem rohrförmigen Körper einstückig ist, und einen äußeren Flanschteil 21B umfasst. Der äußere Flanschteil 21B weist eine Ausnehmung 22 auf, in die der innere Flanschteil 21A passend eingesetzt werden kann. Die Ausnehmung 22 des äußeren Flanschteils 21B und die Umfangsfläche des inneren Flanschteils 21A sind mehreckig, so dass der innere Flanschteil verdrehsicher in dem äußeren Flanschteil sitzt. Der äußere Flanschteil 21B kann wiederum in eine entsprechende mehreckige Ausnehmung 23 in dem Gehäusteteil 1A eingesetzt werden, so dass der äußere Flanschteil verdrehsicher in dem Gehäuse sitzt. Der äußere Flanschteil 21B besteht aus einem elektrisch nicht leitenden Material, beispielsweise ist der äußere Flanschteil eine Isolierscheibe aus Kunststoff.

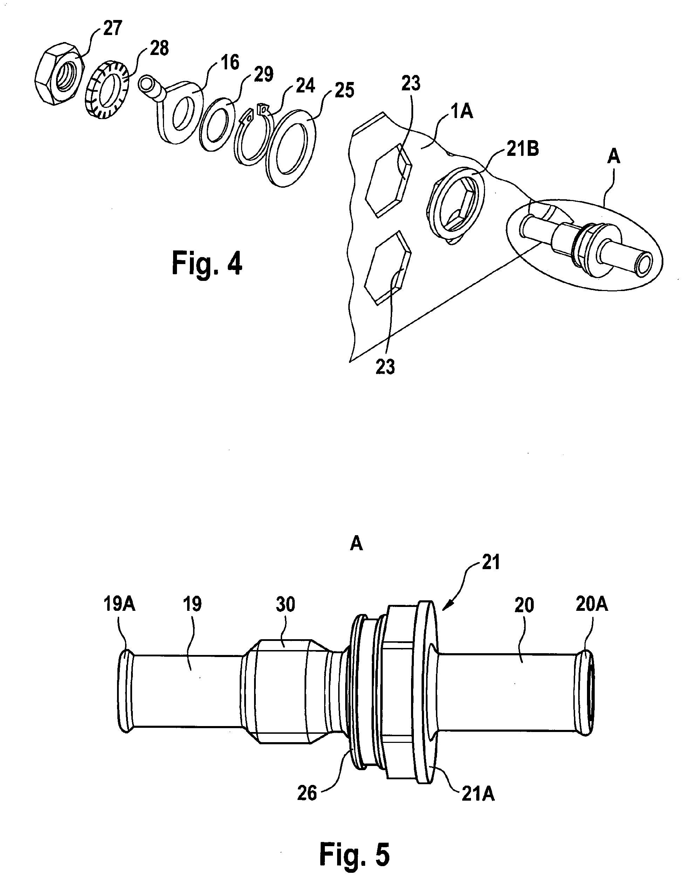

Fig. 4 zeigt eine Explosionsdarstellung, in der die mehreckige Ausnehmung 23 in dem Gehäuseteil 1A erkennbar ist. Fig. 5 zeigt den rohrförmigen Körper 18 mit dem inneren Flanschteil 21A in vergrößerter Darstellung.

Wenn der äußere Flanschteil 21B in die Ausnehmung 23 des Gehäuseteils 1A und der innere Flanschteil 21A in den äußeren Flanschteil 21B eingesetzt sind, stützt sich das Verbindungsstück 13 an dem Gehäuseteil 1A ab. Das Verbindungsstück 13 wird in dem Gehäuseteil 1A mit einem vorzugsweise metallischen Sicherungsring 24 gesichert, der in einer Nut des rohrförmigen Körpers 18 sitzt. Zwischen dem Flansch 21 und dem Sicherungsring 24 befindet sich eine Unterlegscheibe 25 aus einem elektrisch nicht leitenden Material, beispielsweise eine Kunststoffunterlegscheibe. Folglich ist der rohrförmige Körper 18 gegenüber dem Gehäuseteil 1A elektrisch isoliert.

Der Anschlussteil des Verbindungsstücks 13 zum Anschluss des Schutzleiters ist bei dem Ausführungsbeispiel ein Ringkabelschuh 16, der mit dem rohrförmigen Körper 18 verschraubt werden kann. Hierzu weist der rohrförmige Körper 18 einen umlaufenden Ansatz 26 auf, der in axialer Richtung neben dem Sicherungsring 24 angeordnet ist. An den umlaufenden Ansatz 26 schließt sich in axialer Richtung ein Außengewinde 30 an, auf das eine Mutter 27 aufgeschraubt werden kann. Der Ringkabelschuh 16 wird beim Festdrehen der Mutter 27 kraftschlüssig fixiert. Zwischen der Mutter 27 und dem Ringkabelschuh 16 befindet sich eine Fächerscheibe 28 und zwischen dem Ringkabelschuh 16 und dem umlaufenden Ansatz 26 eine Unterlegscheibe 29 aus Metall.

An dem Ringkabelschuh 16 ist das zu dem zentralen Schutzleiteranschluss 15 führende Verbindungskabel 17 angeschlossen. Der Anschluss des Kabels an den Ringkabelschuh kann mit den bekannten Verbindungstechniken erfolgen.

## Patentansprüche

1. Verbindungsstück für die Herstellung einer Flüssigkeitsverbindung von flüssigkeitsführenden Leitungen zur Montage in einer Ausnehmung eines Gehäuseteils eines medizinischen Geräts, insbesondere Dialysegeräts, mit einem in die Ausnehmung (23) des Gehäuseteils (1A) einsetzbaren rohrförmigen Körper (18) aus einem elektrisch leitfähigen Material, dessen Endstücke (19, 20) zum Anschluss der flüssigkeitsführenden Leitungen ausgebildet sind, **dadurch gekennzeichnet, dass** das Verbindungsstück einen Anschlussteil (16) zum Anschluss eines elektrischen Schutzleiters oder Potentialausgleichs aufweist, wobei der Anschlussteil (16) elektrisch leitend mit dem rohrförmigen Körper verbindbar ist oder verbunden ist.

2. Verbindungsstück nach Anspruch 1, **dadurch gekennzeichnet, dass** der Anschlussteil (16) ein Ringkabelschuh ist, der mit dem rohrförmigen Körper (18) verschraubbar ist.

3. Verbindungsstück nach Anspruch 1 oder 2, **dadurch gekennzeichnet, dass** der rohrförmige Körper (18) einen umlaufenden Ansatz (26) aufweist, an den sich ein Außengewinde (30) anschließt.

4. Verbindungsstück nach Anspruch 2, **dadurch gekennzeichnet, dass** der Anschlussteil (16) eine Anschlussfahne ist, die mit dem rohrförmigen Körper (18) elektrisch leitend verbunden ist.

5. Verbindungsstück nach einem der Ansprüche 1 bis 4, **dadurch gekennzeichnet, dass** der rohrförmige Körper (18) zwischen den Endstücken (19, 20) einen Flansch (21) aufweist, mit dem der rohrförmige Körper (18) an dem Gehäuseteil (1A) abstützbar ist, und einen Sicherungsteil (24) aufweist, der an dem rohrförmigen Körper (18) befestigbar ist, so dass der rohrförmige Körper in der Ausnehmung (23) des Gehäuseteils (1A) in axialer Richtung gesichert ist.

6. Verbindungsstück nach Anspruch 5, **dadurch gekennzeichnet, dass** der Flansch (21) des rohrförmigen Körpers (18) einen inneren Flanschteil (21A), der mit dem rohrförmigen Körper einstückig ist, und einen äußeren Flanschteil (21B) aufweist, der eine Ausnehmung (22) aufweist, in die der innere Flanschteil (21A) einsetzbar ist, wobei der rohrförmigen Körper (18) mit dem äußeren Flanschteil an dem Gehäuseteil (1A) abstützbar ist, und der äußere Flanschteil (21B) aus einem elektrisch nicht leitenden Material besteht.

7. Verbindungsstück nach Anspruch 6, **dadurch gekennzeichnet, dass** der innere Flanschteil (21A) in dem äußeren Flanschteil (21B) gegen Verdrehen gesichert ist.

8. Verbindungsstück nach Anspruch 6 oder 7, **dadurch gekennzeichnet, dass** der äußere Flanschteil (21B) eine mehreckige Ausnehmung (22) aufweist, und der innere Flanschteil (21A) eine mehreckige Umfangsfläche aufweist.

9. Verbindungsstück nach einem der Ansprüche 5 bis 7, **dadurch gekennzeichnet, dass** der Sicherungsteil (24) ein Sicherungsring ist, der an dem rohrförmigen Körper (18) befestigbar ist.

10. Verbindungsstück nach Anspruch 9, **dadurch gekennzeichnet, dass** das Verbindungsstück eine Unterlegscheibe (25) aus einem elektrisch nicht leitenden Material aufweist, die zwischen dem Sicherungsring (24) und dem Flanschteil (21) des rohrförmigen Körpers einsetzbar ist.

11. Medizinisches Gerät mit einem Gehäuse (1), das einen Gehäuseteil (1A) mit einer Ausnehmung (23) aufweist, in welche ein Verbindungsstück (13) nach einem der Ansprüche 1 bis 10 eingesetzt ist.

12. Medizinisches Gerät nach Anspruch 11, **dadurch gekennzeichnet, dass** der Gehäuseteil (1A) aus einem elektrisch leitfähigen Material besteht.

13. Medizinisches Gerät nach Anspruch 11 oder 12, **dadurch gekennzeichnet, dass** das medizinische Gerät ein Netzteil (14) mit einem zentralen Schutzleiteranschluss (15) aufweist, und dass der Anschlussteil (16) des Verbindungsstücks (13) mit einem elektrischen Verbindungskabel (17) mit dem zentralen Schutzleiteranschluss (15) elektrisch verbunden ist.

14. Medizinisches Gerät nach einem der Ansprüche 11 bis 13, **dadurch gekennzeichnet, dass** das medizinische Gerät einen Flüssigkeitsteil (9) mit flüssigkeitsführenden Leitungen (11, 12) aufweist, wobei die flüssigkeitsführenden Leitungen (11, 12) an den Endstücken (19, 20) des Verbindungsstücks (13) angeschlossen sind.

15. Medizinisches Gerät nach einem der Ansprüche 11 bis 14, **dadurch gekennzeichnet, dass** das medizinisches Gerät ein Dialysegerät ist, dass einen Dialysierflüssigkeitsteil (9) mit einer zu einem Dialysator (2) führenden Dialysierflüssigkeitszuführleitung (11) und einer von dem Dialysator abgehenden Dialysierflüssigkeitsabführleitung (12) aufweist, wobei ein Abschnitt (11A, 12A) der Dialysierflüssigkeitszuführleitung (11) oder Dialysierflüssigkeitsabführleitung (12) an dem einen Endstück (19) und ein anderer Abschnitt (11B, 12B) der Dialysierflüssigkeitszuführleitung oder Dialysierflüssigkeitsabführleitung an dem anderen Endstück (20) des Verbindungstücks (13) angeschlossen sind.

## Claims

1. Connecting piece for establishing a fluid connection between fluid-conducting lines for installation in a cut-out in a housing part of a medical device, in particular a dialysis unit, comprising a tubular body (18) that is made of an electrically conductive material and can be inserted into the cut-out (23) in the housing part (1A), the end pieces (19, 20) of which body are designed for the attachment of the fluid-conducting lines, **characterised in that** the connecting piece comprises a terminal part (16) for attaching an electrical protective conductor or equipotential bonding system, the terminal part (16) being electrically conductively connectable or connected to the tubular body.

2. Connecting piece according to claim 1, **characterised in that** the terminal part (16) is a ring cable lug that can be screwed to the tubular body (18).

3. Connecting piece according to either claim 1 or claim 2, **characterised in that** the tubular body (18) comprises a circumferential shoulder (26) to which an external thread (30) is attached.

4. Connecting piece according to claim 2, **characterised in that** the terminal part (16) is a terminal lug that is electrically conductively connected to the tubular body (18).

5. Connecting piece according to any of claims 1 to 4, **characterised in that** the tubular body (18) comprises, between the end pieces (19, 20), a flange (21) by means of which the tubular body (18) can be supported on the housing part (1A), and comprises a securing part (24) that can be attached to the tubular body (18) such that the tubular body is secured in the cut-out (23) in the housing part (1A) in the axial direction.

6. Connecting piece according to claim 5, **characterised in that** the flange (21) of the tubular body (18) comprises an inner flange part (21A) that is formed in one piece with the tubular body and an outer flange part (21B) that comprises a cut-out (22) into which the inner flange part (21A) can be inserted, it being possible for the tubular body (18) to be supported on the housing part (1A) by the outer flange part, and the outer flange part (21B) consisting of an electrically non-conductive material.

7. Connecting piece according to claim 6, **characterised in that** the inner flange part (21A) is secured against twisting in the outer flange part (21B).

8. Connecting piece according to either claim 6 or claim 7, **characterised in that** the outer flange part (21B) comprises a polygonal cut-out (22), and the inner flange part (21A) comprises a polygonal peripheral surface.

9. Connecting piece according to any of claims 5 to 7, **characterised in that** the securing part (24) is a securing ring that can be attached to the tubular body (18).

10. Connecting piece according to claim 9, **characterised in that** the connecting piece comprises a washer (25) that is made of an electrically non-conductive material and can be inserted between the securing ring (24) and the flange part (21) of the tubular body.

11. Medical device comprising a housing (1) that comprises a housing part (1A) having a cut-out (23), into which a connecting piece (13) according to any of claims 1 to 10 is inserted.

12. Medical device according to claim 11, **characterised in that** the housing part (1A) consists of an electrically conductive material.

13. Medical device according to either claim 11 or claim 12, **characterised in that** the medical device has a power unit (14) comprising a central protective-conductor connection (15), and **in that** the terminal part (16) of the connecting piece (13) is electrically connected to the central protective-conductor connection (15) by an electrical connection cable (17).

14. Medical device according to any of claims 11 to 13, **characterised in that** the medical device comprises a fluid part (9) having fluid-conducting lines (11, 12), the fluid-conducting lines (11, 12) being attached to the end pieces (19, 20) of the connecting piece (13).

15. Medical device according to any of claims 11 to 14, **characterised in that** the medical device is a dialysis unit that comprises a dialysate part (9) having a dialysate feed line (11) that leads to a dialyser (2) and a dialysate removal line (12) that leads away from the dialyser, one portion (11A, 12A) of the dialysate feed line (11) or dialysate removal line (12) being attached to one end piece (19) and another portion (11B, 12B) of the dialysate feed line or dialysate removal line being attached to the other end piece (20) of the connecting piece (13).

## Revendications

1. Raccord pour l'établissement d'une liaison fluidique entre des conduits de guidage de liquide, destiné à être monté dans un évidement d'une partie de boîtier d'un appareil médical, en particulier d'un appareil de dialyse, avec un corps (18) tubulaire pouvant être inséré dans l'évidement (23) de la partie de boîtier (1A), composé d'un matériau électriquement conducteur, dont les embouts (19, 20) sont réalisés pour le raccordement des conduits de guidage de liquide, **caractérisé en ce que** le raccord présente une partie de raccordement (16) pour le raccordement d'un conducteur de protection ou d'un système de compensation de potentiel électriques, dans lequel la partie de raccordement (16) peut être connectée ou est connectée de manière électriquement conductrice au corps tubulaire.

2. Raccord selon la revendication 1, **caractérisé en ce que** la partie de raccordement (16) est une cosse terminale annulaire, qui peut être vissée au corps (18) tubulaire.

3. Raccord selon la revendication 1 ou 2, **caractérisé en ce que** le corps (18) tubulaire présente un appendice (26) périphérique, auquel se raccorde un filetage extérieur (30).

4. Raccord selon la revendication 2, **caractérisé en ce que** la partie de raccordement (16) est une barrette de raccordement, qui est connectée de manière électriquement conductrice au corps (18) tubulaire.

5. Raccord selon l'une quelconque des revendications 1 à 4, **caractérisé en ce que** le corps (18) tubulaire présente entre les embouts (19, 20) un flasque (21), avec lequel le corps (18) tubulaire peut prendre appui au niveau de la partie de boîtier (1A), et présente une partie de blocage (24), qui peut être fixée au niveau du corps (18) tubulaire de sorte que le corps tubulaire est bloqué dans une direction axiale dans l'évidement (23) de la partie de boîtier (1A).

6. Raccord selon la revendication 5, **caractérisé en ce que** le flasque (21) du corps (18) tubulaire présente une partie de flasque intérieure (21A), qui est d'un seul tenant avec le corps tubulaire, et une partie de flasque extérieure (21B), qui présente un évidement (22), dans lequel la partie de flasque intérieure (21A) peut être insérée, dans lequel le corps (18) tubulaire peut prendre appui avec la partie de flasque extérieure au niveau de la partie de boîtier (1A), et la partie de flasque extérieure (21B) est constituée d'un matériau non conducteur électriquement.

7. Raccord selon la revendication 6, **caractérisé en ce que** la partie de flasque intérieure (21A) est bloquée dans la partie de flasque extérieure (21B) pour empêcher toute rotation.

8. Raccord selon la revendication 6 ou 7, **caractérisé en ce que** la partie de flasque extérieure (21B) présente un évidement (22) polygonal, et la partie de flasque intérieure (21A) présente une surface périphérique polygonale.

9. Raccord selon l'une quelconque des revendications 5 à 7, **caractérisé en ce que** la partie de blocage (24) est une bague de blocage, qui peut être fixée au niveau du corps (18) tubulaire.

10. Raccord selon la revendication 9, **caractérisé en ce que** le raccord présente une rondelle (25) composée d'un matériau non conducteur électriquement, qui peut être insérée entre la bague de blocage (24) et la partie de flasque (21) du corps tubulaire.

11. Appareil médical avec un boîtier (1), qui présente une partie de boîtier (1A) avec un évidement (23), dans lequel est inséré un raccord (13) selon l'une quelconque des revendications 1 à 10.

12. Appareil médical selon la revendication 11, **caractérisé en ce que** la partie de boîtier (1A) est constituée d'un matériau électriquement conducteur.

13. Appareil médical selon la revendication 11 ou 12, **caractérisé en ce que** l'appareil médical présente un bloc d'alimentation (14) avec un raccordement de conducteur de protection (15) central, et que la partie de raccordement (16) du raccord (13) est raccordée électriquement avec un câble de connexion (17) électrique à une borne de conducteur de protection (15) centrale.

14. Appareil médical selon l'une quelconque des revendications 11 à 13, **caractérisé en ce que** l'appareil médical présente une partie de liquide (9) avec des conduits (11, 12) de guidage de liquide, dans lequel les conduits (11, 12) de guidage de liquide sont raccordés aux embouts (19, 20) du raccord (13).

15. Appareil médical selon l'une quelconque des revendications 11 à 14, **caractérisé en ce que** l'appareil médical est un appareil de dialyse, qui présente une partie de liquide de dialyse (9) avec un conduit d'arrivée de liquide de dialyse (11) menant à un dialyseur (2) et un conduit d'évacuation de liquide de dialyse (12) partant du dialyseur, dans lequel une section (11A, 12A) du conduit d'arrivée de liquide de dialyse (11) ou du conduit d'évacuation de liquide de dialyse (12) est raccordée à un embout (19) et une autre section (11B, 12B) du conduit d'arrivée de liquide de dialyse ou du conduit d'évacuation de liquide de dialyse est raccordée à l'autre embout (20) du raccord (13).
